# EUROPEAN PATENT SPECIFICATION

(11) **EP 2 968 183 B1**
(45) Date of publication and mention of the grant of the patent: **09.05.2018**
(21) Application number: 14714141.0
(22) Date of filing: 14.03.2014
(51) Int. Cl.: A61K 9/48, A61K 31/565, A61K 47/18, A61K 47/32, A61K 47/10

(54) **PHARMACEUTICAL SOFT GELATIN CAPSULE DOSAGE FORM**
PHARMAZEUTISCHE WEICHGELATINEKAPSEL-DARREICHUNGSFORM
FORME GALÉNIQUE CAPSULE PHARMACEUTIQUE À ENVELOPPE MOLLE

(30) Priority: 15.03.2013 US 201361794813 P
(43) Date of publication of application: 20.01.2016
(73) Proprietor: Allergan Pharmaceuticals International Limited, Dublin 17 (IE)
(72) Inventor: MULDOON, Brendan, Glengormley Newtownabbey BT6 7DL (IE); MCCULLAGH, Stephen, Belfast BT6 0LL (IE)
(74) Representative: FRKelly
(86) International application number: PCT/US2014/027093
(87) International publication number: WO 2014/152226

(56) References cited:
- EP-A1- 0 770 384
- US-A1- 2004 131 670
- US-A1- 2007 098 783

## Description

### BACKGROUND OF THE INVENTION

### Field of the Invention

This invention relates to a pharmaceutical soft gelatin capsule dosage form that has a stable dissolution profile over the time of storage so that an active ingredient may be delivered to the desired site in a manner that provides for consistent dosing.

### DESCRIPTION OF RELATED ART

Soft gelatin pharmaceutical formulations have several advantages, such as, they are easy to swallow, they mask the odors and unpleasant tastes, and once swallowed, they release their contents very quickly. However, soft gelatin capsules have been known to have a decrease in dissolution during storage, which eventually may retard or deleteriously impact drug release. The decrease in dissolution has typically been attributed to the crosslinking of gelatin in the capsule shell resulting in a pellicle formation. Pellicle formation can be minimized by various techniques such as using excipients in the capsule fill with low grade peroxides and aldehydes, or using gelatin grades less prone to pellicle formation to minimize the formation of crosslinking agents. The manufacturing process can also be optimized, for example, by storing fill under nitrogen, controlling the temperature and humidity of manufacturing environment, minimizing the temperature and heat exposure time of heating processes, testing excipients for formaldehyde or low molecular weight aldehydes levels, and using moisture and/or light resistant packaging.

Applicants have found that even when steps are taken to minimize pellicle formation, soft gelatin capsules containing ionic components such as polyacrylic acid in the fill can exhibit unstable dissolution profiles after storage. It is believed, without being bound by theory, that the polyacrylic acid contained in the fill of the soft gelatin capsule interacts with the gelatin in the shell, inhibiting rupture and thus altering the dissolution profile.

Singh et al., "Gelatin-Containing Formulations: Changes in Dissolution Characteristics," Encyclopedia of Pharmaceutical Technology, 2003, describes various mechanisms for pellicle formation in gelatin-based dosage forms and the solutions proposed to overcome the problem.

U.S. Patent Application Publication No. 2004/0131670 describes a composition suitable for preparing a pharmaceutical capsule shell comprising gelatin and an amine agent that comprises at least one pharmaceutically acceptable primary amine or a secondary amine. The amine agent is present in an amount effective to inhibit cross-linking of the gelatin and/or pellicle formation in a capsule shell prepared from the composition. There is no recognition of preparing a soft gelatin capsule for vaginal administration, let alone a soft gelatin capsule for any administration, that overcomes the problems associated with polyacrylic acid fills in soft gelatin capsules.

U.S. Patent No. 5,874,106 describes a method of reducing crosslinking in gelatin capsules by the incorporation of an amino acid and a carboxylic acid into the capsule fill. It is asserted that crosslinking will likely have a greater impact on *in vitro* dissolution testing than on *in vivo* bioavailability of drugs formulated in gelatin capsules, but this statement is made in the context of gelatin capsules for oral administration. This, however, is not true for a gelatin capsule formulation that is administered vaginally where the dissolution profile of the drug may significantly impact its adsorption by the vaginal epithelial tissue. In addition, there is no recognition of the dissolution problem that occurs when the gelatin capsule contains a polyacrylic acid fill.

European Patent Publication No. 0770384 discloses anhydrous solid pharmaceutical composition for vaginal use, comprising at least one active ingredient in a mixture with conventional carriers and excipients, containing one or more muco-adhesive polymers dispersed in the carrier.

Accordingly, there remains a need for a pharmaceutical soft gelatin capsule dosage form that can deliver a low dose drug, such as an estrogen, and provide a reproducible and constant dosage even after the capsule has been in storage up to one, or more preferably, two years.

### SUMMARY OF THE INVENTION

The present invention is directed to a pharmaceutical soft gelatin capsule dosage form comprising:a shell comprising gelatin and a plasticizer; and a fill comprising at least one pharmaceutically active ingredient, polyethylene glycol, polyacrylic acid, a neutralizing agent, and water,wherein the neutralizing agent is a primary amine or a secondary amine and is present in an amount necessary to provide a soft gelatin dosage form having stable dissolution after storage, and wherein the at least one active ingredient is selected from the group consisting of estradiol, its salts, esters and hydrates. In an embodiment, stable dissolution after storage is achieved when the soft gelatin capsule stored for one month at 40°C and a relative humidity of 75% had less than about 30% change in dissolution after one month storage.

The dissolution is measured using the Dissolution Method described herein.

In one embodiment, the pharmaceutical soft gelatin capsule dosage form of the invention is used for vaginal administration.

The active ingredient is estradiol, or a salt, ester, or hydrate thereof.

### BRIEF DESCRIPTION OF THE DRAWINGS

Fig. 1 is a plot depicting the results of the assay of estradiol for Examples 2, 3 and 5 of the present invention and for Comparative Example 4.
Fig. 2 is a plot depicting the results of the dissolution at 60 minutes for Examples 2, 3, and 5 of the present invention and for Comparative Example 4.

### DETAILED DESCRIPTION OF THE INVENTION

An embodiment of the present invention is directed to a pharmaceutical soft gelatin capsule dosage form comprising: (a) a shell comprising gelatin and a plasticizer; and (b) a fill comprising at least one pharmaceutically active ingredient, polyethylene glycol, polyacrylic acid, a neutralizing agent, and water, wherein the neutralizing agent is a primary amine or a secondary amine. The neutralizing agent is present in an amount necessary to provide a pharmaceutical soft gelatin capsule dosage form having stable dissolution after storage as measured according to Dissolution Method described herein. In an embodiment, stable dissolution after storage is achieved when the soft gelatin capsule stored for one month at 40°C and a relative humidity of 75% had less than about 30%, preferably less than 25%, more preferably less 20% change in dissolution after one month storage.

The pharmaceutical soft gelatin capsule dosage form of the invention may be administered orally or vaginally. A particular embodiment includes the pharmaceutical soft gelatin capsule dosage form wherein the pharmaceutically active ingredient is estrogen and which is administered vaginally.

As used herein "pharmaceutically acceptable" means the component must be considered appropriate for oral or vaginal administration to humans. In one embodiment, the component must be considered appropriate for application to the vaginal environment.

Soft gelatin capsules are well known and are often described as softgels. They comprise a one-piece, hermetically sealed gelatin-based shell containing a solution, a suspension, or a semisolid which is referred to as the fill formulation, fill material or fill. The gelatin bloom strength in the soft gelatin capsule is typically about 150 to about 200. Exemplary manufacturers of softgels include Catalent Pharma Solutions, Somerset, N.J., Pharmagel Engineering spa, Lodi, Italy, and Soft Gel Technologies Inc., Commerce, CA. The soft gelatin capsule of the invention is a pharmaceutical dosage form that comprises a gelatin-based shell and a fill.

In an embodiment of the present invention, the shell may comprise gelatin and a plasticizer. The shell may optionally include an opacifier and/or dyes. Gelatin is obtained by the partial hydrolysis of collagen derived from the skin, white connective tissue and bones of animals including cattle, pigs and fish. It mainly consists of water soluble proteins (84-90 % w/w) along with mineral salts (1-2% w/w) and water (8-15% w/w). The protein fraction contains amino acids linked by amide bonds in a polypeptide chain.

Collagen is a fibrous protein and the main constituent of animal skin, bone and connective tissue. It consists of a triple helix of three polypeptide chains with a molecular weight of approximately 300,000 Da. Denaturation involves breaking of the hydrogen bonds to destabilize the collagen helix resulting in a marked decrease in the molecular weight and the intrinsic viscosity. Hydrolysis of collagen by boiling bones or skins in water results in a low yield of impure gelatin with poor physical properties. Therefore, commercial manufacture of gelatin involves initial removal of contaminants before thermal denaturing with the aid of either a dilute acid to result in Type A gelatin or a dilute alkali to result in Type B gelatin. Gelatin is amphoteric in nature with its isoelectric points ranging from 6.0 to 9.0 for Type A gelatin and from 4.7 to 5.3 for Type B gelatin. It is believed that the alkaline hydrolysis causes a greater degree of deamidation of the asparagine and glutamine amino acids in collagen, resulting in a larger number of free carboxylic acid compared to acid hydrolysis. Examples of suitable Type A gelatin include without limitation acid bone gelatin. Examples of suitable Type B gelatin include without limitation lime bone gelatin.

The gelatin-based soft gelatin capsule will generally contain water in an amount of about 1% to about 25 %, more preferably about 1% to about 15%, still more preferably about 5% to about 10% by weight of the gelatin shell after fill has been encapsulated and water has migrated from the capsule to the fill.

In a preferred embodiment, gelatin is present in an amount of about 35% to about 85%, more preferably about 40% to about 80% by weight of the gelatin shell.

In an embodiment of the present invention, any pharmaceutically acceptable plasticizer can be used in the shell. Non-limiting examples of suitable plasticizer include polyhydric alcohols such as sorbitol, glycerin, mannitol, xylitol, and sorbitan; dialkylphthalates; lower alkyl citrates wherein the lower alkyl has 1-6 carbon atoms; glycols and polyglycols including polyethylene glycols with a molecular weight range of about 200 to about 2,000, methoxyl-propylene-glycol, and 1,2-propylene glycol; esters of polyhydroxy-alcohols such as mono-, di-, and tri-acetate of glycerol; ricinoleic acid and esters thereof; and mixtures of the above.

In a preferred embodiment, plasticizer is present in an amount of about 10% to about 60%, more preferably about 20% to about 55%, still more preferably about 30% to about 50% by weight of the gelatin shell.

The fill includes at least one pharmaceutically active ingredient, polyethylene glycol, polyacrylic acid, a neutralizing agent, and water, as defined in claim 1. The fill does not contain ingredients in an amount that would not be pharmaceutically acceptable for oral or vaginal administration.

Disclosed examples of suitable pharmaceutically active ingredient include steroids and low dose non-steroidal compounds, their pharmaceutically acceptable salts, esters, hydrates, prodrugs and derivatives. Disclosed examples of suitable low dose non-steroidal compounds include darifenacin, udenafil and bisphosphonate compounds like risedronate, alendronate, etidronate, ibandronate, clodronate, and zoledronate. Disclosed active ingredients include an estrogenic or progestogenic compound such as, estradiol, ethinyl estradiol, norethindrone acetate, etonogestrel, their pharmaceutically acceptable salts, esters, hydrates, prodrugs and derivatives, and mixtures thereof. The compositions according to the present invention comprise a compound selected from estradiol, its salts, esters or hydrates.

The phrase "pharmaceutically acceptable salt" of a compound as used herein means a salt that is pharmaceutically acceptable and that possesses the desired pharmacological activity of the parent compound. Pharmaceutically acceptable salts include salts of acidic or basic groups present in a compound of the invention. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate (i.e., 1,1'-methylene-bis-(2-hydroxy-3-naphthoate)) salts. Suitable base salts include, but are not limited to, aluminum, calcium, lithium, magnesium, potassium, sodium, zinc, and diethanolamine salts.

The term "ester", as used herein, refers to an organic compound made by replacing the hydrogen of an acid by an alkyl, e.g., C₁ to C₆ alkyl, or other organic group. Various esters are well known in the art. Nonlimiting examples of esters include formate, acetate, propionate, acetyl glycolate, and butyrate.

The term "hydrate", as used herein, refers to a compound formed by the addition of water. The hydrates may be obtained by any known method in the art by dissolving the compounds in water and recrystallizing them to incorporate water into the crystalline structure. Nonlimiting examples of hydrates include hemihydrate, monohydrate, dehydrate, trihydrate, and pentahydrate.

The term "prodrug", as used herein, refers to an inactive precursor of a drug, converted into its active form in the body by normal metabolic processes. Various forms of prodrugs are well known in the art.

In one embodiment, the pharmaceutically active ingredient is present in the soft gelatin capsule of the present invention in an amount of about 0.01µg to about 500 mg, depending on the desired dosage of the active ingredient.

In an embodiment, when the pharmaceutically active ingredient is estrogen, it is included in the pharmaceutical soft gelatin capsule dosage form of the present invention in an amount ranging from about 0.00001% to about 2%, more preferably from about 0.00015% to about 0.0075%, still more preferably about 0.003% by weight of the pharmaceutical capsule fill.

The at least one active ingredient is estrogen and the estrogen is 17β-estradiol or salts, esters or prodrugs thereof. Other suitable estrogens include those described in each of U.S. Patent Nos. 7,067,504, 7,067,505, and 7,795,241, and U.S. Patent Application Publication Nos. 2007/0015741 and 2007/0004694.

The at least one active ingredient is selected from the group consisting of estradiol, its salts, esters, and hydrates. In a preferred embodiment, estrogen is 17β-estradiol. Pharmaceutically acceptable salts of 17β-estradiol are well known and include, without limitation, 17β-estradiol hydrochloride salt, β-estradiol 17-(P-D-glucuronide) sodium salt and β-estradiol 3-(P-D-glucuronide) 17-sulfate dipotassium salt. Esters of 17β-estradiol are also well known and include, without limitation, estradiol-3-acetate, estradiol-17-acetate, estradiol-3,17-diacetate, estradiol-3,17-valerate, estradiol-3-valerate, estradiol-17-valerate, estradiol 3-benzoate, estradiol cypionate, estradiol dipropionate, and estradiol enantate. Hydrates of 17β-estradiol are also well known and include, without limitation, the hemihydrate. Prodrugs of 17β-estradiol are also well known and include, without limitation, the prodrug described in U.S. Patent No. 7,067,505. In a preferred embodiment, the 17β-estradiol is 17β-estradiol hemihydrate.

In an embodiment of the present invention, the polyethylene glycol has a molecular weight range of about 200 to about 2,000. In a preferred embodiment, the polyethylene glycol is PEG 400 or PEG 600.

Polyacrylic acid of the present invention may be homopolymers of acrylic acid, crosslinked with an allyl ether pentaerythritol, allyl ether of sucrose, allyl ether of propylene or divinyl glycol. Non-limiting examples of suitable polyacrylic acid include polycarbophil and carbomer copolymer type A (e.g., commercially available under the tradename Pemulen™ TR-2).

The inventors of the present invention found a decrease in the dissolution of soft gelatin capsules during storage. However, the various known techniques described above to minimize pellicle formation did not help alleviate the problem. The inventors then concluded that the decrease in dissolution was not caused by pellicle formation and hypothesized that it could be caused by the interaction of gelatin with anionic polymer in the fill.

As previously noted, while not wishing to be bound by theory, it was discovered that because polyacrylic acid such as polycarbophil in the fill is an anionic polymer, it interacted with the gelatin to result in the formation of an insoluble mass, which reduces the dissolution stability of the soft gelatin capsule dosage form. It was further theorized that this interaction could be minimized through the use of a neutralizing agent. Other anionic polymers in the fill besides polyacrylic acid also interact with gelatin to result in the formation of an insoluble mass, which reduces the dissolution stability of the soft gelatin capsule dosage form and the present invention can also help minimize this reduction in the dissolution stability. Non-limiting examples of such anionic polymers include poly(methyl vinyl ether/maleic anhydride) (Gantrez)#, carbomer, carboxymethylcellulose calcium, carboxymethylcellulose sodium and alginates, such as calcium alginate, potassium alginate, sodium alginate, and alginic acid.

In an embodiment of the present invention, the neutralizing agent can be a primary amine or a secondary amine, such as straight and branched chain C₁-C₆ alkyl primary and secondary amines. Non-limiting examples of suitable primary and secondary amines include diisopropanolamine, phenylamine, glutamine, hydroxylamine chloride, p-amino benzoic acid, and amino acids. Non-limiting examples of suitable amino acids include glycine and lysine. More preferably, the neutralizing agent is alkanolamine. Even more preferably, the neutralizing agent is propanolamine. Most preferably, the neutralizing agent is diisopropanolamine.

The fill may optionally include antioxidants, buffering agents, or combinations thereof. Non-limiting examples of suitable antioxidants include tocopherol, butylated hydroxytoluene, butylated hydroxyanisole, dodecyl gallate, octyl gallate, propyl gallate, ascorbyl palmitate, sodium ascorbate and thymol. Non-limiting examples of suitable buffering agents include citric acid, benzoic acid, fumaric acid, and maleic acid.

The inventors have found that the interaction of gelatin in the shell with polyacrylic acid, for example, polycarbophil, in the fill is minimized by neutralizing the polyacrylic acid, for example, polycarbophil, with bases such as primary and secondary amines. As will be seen from the examples and comparative examples below, fill formulations containing polycarbophil displayed reduced dissolution when encapsulated with Type A gelatin (for example, acid bone gelatin). This is believed to be due to an interaction between the anionic polymer and the amphoteric gelatin shell forming an insoluble mass. In contrast, encapsulation with Type B gelatin (for example, lime bone gelatin) resulted in improved release. This is believed to be due to the presence of fewer amide groups in the gelatin for polycarbophil to interact with. Lower amounts of neutralizing agent is sufficient to prevent precipitation with aqueous solutions of Type B gelatin, while a similar effect can be obtained with increased amounts of neutralizing agent for solutions of Type A gelatin. The inventors also found that the type of amine in the fill affects the decrease in dissolution. In particular, a fill formulation with a tertiary amine showed a greater reduction in dissolution than a primary amine or a secondary amine. This is believed to be due to the difference in the structures of these amines; unlike primary and secondary amines, tertiary amines do not have a proton available on the amine nitrogen to donate in order to react with the -COOH group on the polycarbophil molecule.

The neutralizing agent is present in an amount necessary to provide a soft gelatin dosage form having stable dissolution after storage. In a preferred embodiment, neutralizing agent is present in an amount of about 0.050% to about 0.500%, more preferably about 0.075% to about 0.400%, still more preferably about 0.100% to about 0.300% by weight of the total weight of the fill.

In a particularly preferred embodiment of the invention it was found that the dissolution of the pharmaceutical soft gelatin capsule dosage form stored at 40°C and a relative humidity of 75% changed less than 30%, preferably less than 25% and most preferably less than 20% after one month storage.

The dissolution of the pharmaceutical soft gelatin capsule dosage form was measured by the following Dissolution Method.

### Dissolution Method

The dissolution was measured using a USP Apparatus 2 with paddles as the dissolution apparatus, a dissolution medium volume of 500 ml of 0.5% hexadecyl trimethyl ammonium bromide surfactant in water, a paddle speed of 100 rpm and a temperature of 37±0.5 °C. As used herein the dissolution was measured on the basis of the release of the active after 60 minutes. A different dissolution method could be employed, i.e., different media, paddle speed, temperature or time to measure, so long as the test was consistently used between start time and measurement time (e.g., 1, 2, 3, 4, 5, 6, etc. months) after comparison to the results achieved using the Dissolution Method specified herein to determine the percent change over time for the inventive formulation.

Specific embodiments of the invention will now be demonstrated by reference to the following examples. It should be understood that these examples are disclosed by way of illustrating the invention and should not be taken in any way to limit the scope of the present invention.

### EXAMPLES

In order to study the effect of various parameters on dissolution of the pharmaceutical soft gelatin capsule dosage form, five different fill gels were selected for formulation in the pharmaceutical soft gelatin capsule dosage form. The composition of the gelatin shells were also varied and were selected from acid bone and lime bone (HLX) gelatin shells. In addition to gelatin, the shell comprised of sorbitol special/glycerin blend A810, which is a blend of 1,4-sorbitan, sorbitol and mannitol (sorbitol sorbitan solution NF) and glycerin USP. Water is used in the manufacture of the gel material up to approximately 40% by weight of wet gel mass solution, however, by the end of the capsule manufacturing process, which involved a number of drying steps, capsules typically have approximately 3% to 10% water by weight of soft gelatine capsule. The compositions of the various fill formulations according to the present invention that were studied are set forth in Table 1.

**TABLE 1**

| **Fill Component** | **% w/w** | | |
|---|---|---|---|
| Formulations with Acid Bone Gelatin Shell | **Example 1** | **Example 2** | **Example 3** |
| Formulations with Lime Bone Gelatin Shell | **Example 4** | **Example 5** | **Example 6** |
| Estradiol | 0.00147 | 0.00147 | 0.00147 |
| PEG 400 | 77.48 | 77.30 | 77.63 |
| Propylene Glycol | 14.00 | 14.00 | 14.00 |
| Polycarbophil | 1.00 | 1.00 | - |
| Pemulen TR-2 | - | - | 0.75 |
| Deionized Water | 7.50 | 7.50 | 7.50 |
| DL-a-Tocopherol | - | - | 0.10 |
| Glycine | 0.02 | - | 0.02 |
| Diisopropanolamine | - | 0.20 | - |
| Total | 100 | 100 | 100 |

### COMPARATIVE EXAMPLES

Comparative fill formulations with trolamine as the neutralizing agent were also studied and their compositions are set forth in Table 2. The shell composition was the same as for the examples above.

**TABLE 2**

| **Fill Component** | **% w/w** |
|---|---|
| Formulations with Acid Bone Gelatin Shell | **Comparative Example 1** |
| Formulations with Lime Bone Gelatin Shell | **Comparative Example 2** |
| Estradiol | 0.00147 |
| PEG 400 | 77.55 |
| Propylene Glycol | 14.00 |
| Polycarbophil | - |
| Pemulen TR-2 | 0.75 |
| Deionized Water | 7.50 |
| DL-a-Tocopherol | - |
| Glycine | - |
| Trolamine | 0.20 |
| Diisopropanolamine | - |
| Total | 100 |

### COMPARATIVE EXAMPLES

Comparative fill formulations without a neutralizing agent were also studied and their compositions are set forth in Table 3. The shell composition was the same as for the examples above.

**TABLE 3**

| **Fill Component** | **% w/w** |
|---|---|
| Formulations with Acid Bone Gelatin Shell | **Comparative Example 3** |
| Formulations with Lime Bone Gelatin Shell | **Comparative Example 4** |
| Estradiol | 0.00147 |
| PEG 400 | 77.50 |
| Propylene Glycol | 14.00 |
| Polycarbophil | 1.00 |
| Pemulen TR-2 | - |
| Deionized Water | 7.50 |
| DL-a-Tocopherol | - |
| Glycine | - |
| Trolamine | - |
| Diisopropanolamine | - |
| Total | 100 |

Full dissolution profiles of the pharmaceutical soft gelatin capsule dosage forms were obtained for the above formulations using the method described above. The dissolution results (% LC) at 60 minutes are set forth in Table 4.

**TABLE 4**

| **Formulation** | **T=0** | **T=2wk** | **T=1M** | **T=2M** | **T=3M** | **T=3M** |
|---|---|---|---|---|---|---|
| | | **40°C/75%RH (%RSD)** | | | | **25°C/60% RH (%RSD)** |
| Example 1 | 83 (18.1) | 54 (4.4) | 27 (8.1) | 13 (10.4) | 10 (4.7) | 63 (2.8) |
| Example 2 | 89 (1.0) | 80 (5.5) | 71 (21.7) | 26 (87.7) | 13 (17.8) | 90 (3.8) |
| Example 3 | 92 (2.2) | 94 (1.4) | 85 (4.5) | 87 (6.2) | 57 (35.7) | 94 (0.8) |
| Comparative Example 1 | 93 (2.7) | 45 (31.1) | 44 (39.8) | 8 (8.2) | 4 (8.1) | 32 (28.1) |
| Comparative Example 3 | 57 (8.9) | 44 (8.4) | 19 (3.7) | 12 (1.8) | - | 47 (3.3) |
| | | | | | | |
| Example 4 | 95 (3.7) | 37 (6.5) | 38 (2.8) | 36 (2.4) | 39 (11.4) | - |
| Example 5 | 96(1.4) | 95 (1.4) | 80 (9.5) | 77 (18.1) | 20 (73.1) | 94 (0.3) |
| Example 6 | 95 (1.0) | 64 (24.2) | 46 (9.5) | 56 (11.2) | 47 (26.5) | 50 (18.8) |
| Comparative Example 2 | 88 (9.4) | 92 (4.2) | 68 (18.6) | 27 (7.8) | - | - |
| Comparative Example 4 | 95 (0.2) | 95 (0.5) | 84 (8.2) | 38 (67.1) | 24 (10.9) | 95 (0.1) |

As seen in Table 4, all formulations showed a significant decrease in dissolution upon storage at 40°C/75%RH. After 3 months at 25°C/60%RH, the decrease in dissolution was not significant for a number of formulations, such as Examples 2, 3 and 5 as well as Comparative Example 4. These formulations were analyzed further. In addition, the fill formulation containing diisopropanolamine maintained dissolution after 3 month at ambient laboratory conditions when encapsulated in acid bone (Example 2) and lime bone (Example 5). It was also observed that the decrease in dissolution for the fill formulation containing trolamine (Comparative Example 1) was comparable to that of the fill formulation without any neutralizing agent when encapsulated in acid bone (Comparative Example 3). However, the decrease in dissolution for the fill formulation containing trolamine (Comparative Example 2) was greater than that of the fill formulation without any neutralizing agent when encapsulated in lime bone (Comparative Example 4).

Assay was performed by dissolving seven capsules in water and acetonitrile, sonicating for a total of 60 minutes and using HPLC analysis with fluorescence detector. Dissolution was measured according to Dissolution Method described above. Table 5 summarizes results obtained for assay and dissolution of Examples 2, 3 and 5 as well as Comparative Example 4 samples stored at ambient laboratory conditions for 20 months.

**TABLE 5**

| **Formulation** | **Assay (n=2, average %LC) T=20 M @ Ambient** | **Dissolution (n=6, average %LC 60 min)** |
|---|---|---|
| Example 2 | 99.2 | 75 (23.0% RSD) |
| Example 3 | 98.6 | 89 (9.1% RSD) |
| Example 5 | 100.6 | 93 (1.9% RSD) |
| Comparative Example 4 | 98.1 | 46 (53.5 %RSD) |

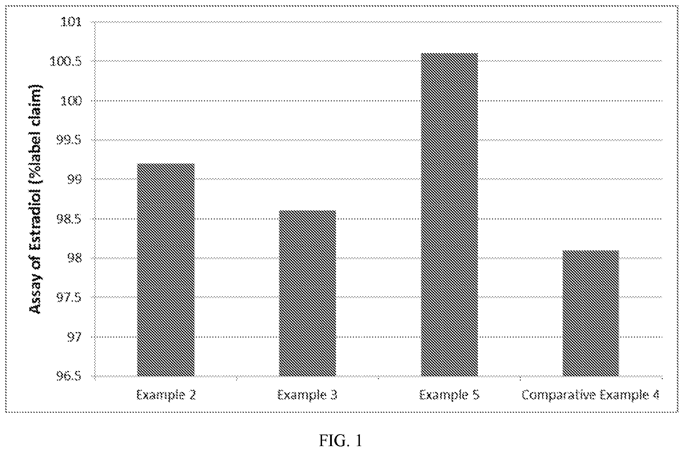

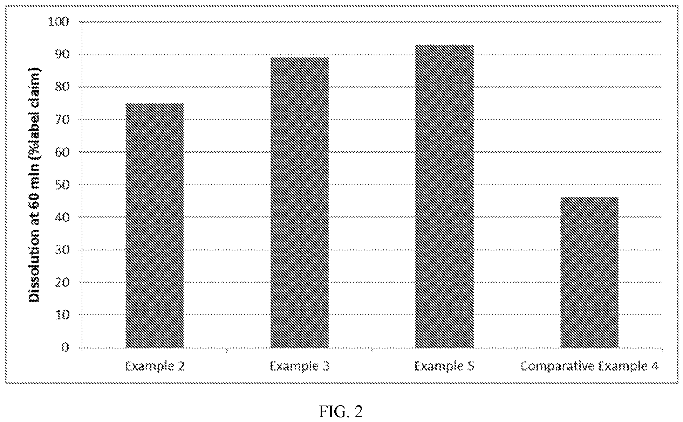

The results of Table 5 are also shown as bar graphs in Fig. 1 and Fig. 2.

As seen in Table 5, the fill formulation containing diisopropanolamine (Example 5) encapsulated in lime bone had the highest dissolution after 20 months at ambient laboratory conditions. In contrast, the fill formulation containing no neutralizing agent (Comparative Example 4) encapsulated in lime bone had the lowest dissolution after comparable testing conditions.

While the invention has been described above with reference to specific embodiments thereof, it is apparent that many changes, modifications, and variations can be made without departing from the inventive concept disclosed herein. Accordingly, it is intended to embrace all such changes, modifications, and variations that fall within the scope of the appended claims.

## Claims

1. A pharmaceutical soft gelatin capsule dosage form comprising:
a shell comprising gelatin and a plasticizer; and
a fill comprising at least one pharmaceutically active ingredient, polyethylene glycol, polyacrylic acid, a neutralizing agent, and water,
wherein the neutralizing agent is a primary amine or a secondary amine and is present in an amount necessary to provide a soft gelatin dosage form having stable dissolution after storage, and wherein the at least one active ingredient is selected from the group consisting of estradiol, its salts, esters and hydrates.

2. The pharmaceutical soft gelatin capsule dosage form of claim 1, wherein stable dissolution after storage is achieved when the soft gelatin capsule stored for one month at 40°C and a relative humidity of 75% had less than 30% change in dissolution after one month storage.

3. The pharmaceutical soft gelatin capsule dosage form of claim 1 or 2, wherein the pharmaceutical soft gelatin capsule dosage form is for oral or vaginal administration.

4. The pharmaceutical soft gelatin capsule dosage form of claim 1 or 2, wherein the soft gelatin capsule is for vaginal administration.

5. The pharmaceutical soft gelatin capsule dosage form of any one of claims 1 to 4, wherein the polyacrylic acid is polycarbophil.

6. The pharmaceutical soft gelatin capsule dosage form of any one of claims 1 to 5, wherein the neutralizing agent is selected from the group consisting of C₁-C₆ alkyl primary amines and C₁-C₆ alkyl secondary amines.

7. The pharmaceutical soft gelatin capsule dosage form of any one of claims 1 to 5, wherein the neutralizing agent is selected from the group consisting of diisopropanolamine, phenylamine, glutamine, hydroxylamine chloride, p-amino benzoic acid, and amino acids selected from glycine and lysine.

8. The pharmaceutical soft gelatin capsule dosage form of any one of claims 1 to 5, wherein the neutralizing agent is an alkanolamine.

9. The pharmaceutical soft gelatin capsule dosage form of claim 8, wherein the alkanolamine is propanolamine.

10. The pharmaceutical soft gelatin capsule dosage form of claim 8, wherein the alkanolamine is diisopropanolamine.

11. The pharmaceutical soft gelatin capsule dosage form of any one of claims 1 to 10, wherein the amount of the neutralizing agent is 0.05% to 0.5% by weight of the total weight of the fill.

12. The pharmaceutical soft gelatin capsule dosage form of claim 11, wherein the amount of the neutralizing agent is present in an amount of 0.075% to 0.400% by weight of the total weight of the fill.

13. The pharmaceutical soft gelatin capsule dosage form of claim 11 or 12, wherein the amount of the neutralizing agent is present in an amount of 0.100% to 0.300% by weight of the total weight of the fill.

14. The pharmaceutical soft gelatin capsule dosage form of claim 11, wherein the neutralizing agent is diisopropanolamine.

15. The pharmaceutical soft gelatin capsule dosage form of claim 14, wherein the amount of diisopropanolamine is 0.2% by weight of the total weight of the fill.

## Patentansprüche

1. Pharmazeutische Weichgelatinekapsel-Dosierungsform, die Folgendes umfasst:
eine Hülle, die Gelatine und einen Weichmacher umfasst; und
eine Füllung, die mindestens einen pharmazeutischen Wirkstoff, Polyethylenglycol, Polyacrylsäure, ein Neutralisationsmittel und Wasser umfasst,
wobei das Neutralisationsmittel ein primäres Amin oder ein sekundäres Amin ist und in einer Menge vorliegt, die zur Bereitstellung einer Weichgelatine-Dosierungsform notwendig ist, die eine stabile Auflösung nach Lagerung aufweist, und wobei der mindestens eine Wirkstoff aus der Gruppe ausgewählt ist, die aus Estradiol, dessen Salzen, Estern und Hydraten besteht.

2. Pharmazeutische Weichgelatinekapsel-Dosierungsform nach Anspruch 1, wobei eine stabile Auflösung nach Lagerung erreicht wird, wenn die Weichgelatinekapsel, die für einen Monat bei 40 °C und einer relativen Feuchte von 75 % gelagert wurde, eine Veränderung von weniger als 30 % der Auflösung nach einem Monat Lagerung aufwies.

3. Pharmazeutische Weichgelatinekapsel-Dosierungsform nach Anspruch 1 oder 2, wobei die pharmazeutische Weichgelatinekapsel-Dosierungsform für die orale oder vaginale Verabreichung vorgesehen ist.

4. Pharmazeutische Weichgelatinekapsel-Dosierungsform nach Anspruch 1 oder 2, wobei die Weichgelatinekapsel für die vaginale Verabreichung vorgesehen ist.

5. Pharmazeutische Weichgelatinekapsel-Dosierungsform nach einem der Ansprüche 1 bis 4, wobei die Polyacrylsäure Polycarbophil ist.

6. Pharmazeutische Weichgelatinekapsel-Dosierungsform nach einem der Ansprüche 1 bis 5, wobei das Neutralisationsmittel aus der Gruppe ausgewählt ist, die aus primären C₁-C₆-Alkylaminen und sekundären C₁-C₆-Alkylaminen besteht.

7. Pharmazeutische Weichgelatinekapsel-Dosierungsform nach einem der Ansprüche 1 bis 5, wobei das Neutralisationsmittel aus der Gruppe ausgewählt ist, die aus Folgenden besteht: Diisopropanolamin, Phenylamin, Glutamin, Hydroxylaminchlorid, p-Aminobenzoesäure und Aminosäuren, die aus Glycin und Lysin ausgewählt sind.

8. Pharmazeutische Weichgelatinekapsel-Dosierungsform nach einem der Ansprüche 1 bis 5, wobei das Neutralisationsmittel ein Alkanolamin ist.

9. Pharmazeutische Weichgelatinekapsel-Dosierungsform nach Anspruch 8, wobei das Alkanolamin Propanolamin ist.

10. Pharmazeutische Weichgelatinekapsel-Dosierungsform nach Anspruch 8, wobei das Alkanolamin Diisopropanolamin ist.

11. Pharmazeutische Weichgelatinekapsel-Dosierungsform nach einem der Ansprüche 1 bis 10, wobei die Menge des Neutralisationsmittels 0,05 Gewichts-% bis 0,5 Gewichts-% des Gesamtgewichts der Füllung beträgt.

12. Pharmazeutische Weichgelatinekapsel-Dosierungsform nach Anspruch 11, wobei die Menge des Neutralisationsmittels in einer Menge von 0,075 Gewichts-% bis 0,400 Gewichts-% des Gesamtgewichts der Füllung vorliegt.

13. Pharmazeutische Weichgelatinekapsel-Dosierungsform nach Anspruch 11 oder 12, wobei die Menge des Neutralisationsmittels in einer Menge von 0,100 Gewichts-% bis 0,300 Gewichts-% des Gesamtgewichts der Füllung vorliegt.

14. Pharmazeutische Weichgelatinekapsel-Dosierungsform nach Anspruch 11, wobei das Neutralisationsmittel Diisopropanolamin ist.

15. Pharmazeutische Weichgelatinekapsel-Dosierungsform nach Anspruch 14, wobei die Menge von Diisopropanolamin 0,2 Gewichts-% des Gesamtgewichts der Füllung beträgt.

## Revendications

1. Forme de dosage en capsule de gélatine molle pharmaceutique, comprenant :
une enveloppe comprenant de la gélatine et un plastifiant ; et
un remplissage comprenant au moins un ingrédient pharmaceutiquement actif, du polyéthylène glycol, de l'acide polyacrylique, un agent neutralisant, et de l'eau ;
où l'agent neutralisant est une amine primaire ou une amine secondaire, et est présent selon une quantité nécessaire pour fournir une forme de dosage de gélatine molle ayant une dissolution stable après stockage, et où le au moins un ingrédient actif est choisi dans le groupe constitué par l'estradiol, ses sels, esters et hydrates.

2. Forme de dosage en capsule de gélatine molle pharmaceutique selon la revendication 1, où la dissolution stable après stockage est obtenue lorsque la capsule de gélatine molle stockée pendant un mois à 40°C et une humidité relative de 75% avait moins de 30% de changement de dissolution après un mois de stockage.

3. Forme de dosage en capsule de gélatine molle pharmaceutique selon la revendication 1 ou 2, où la forme de dosage en capsule de gélatine molle pharmaceutique est destinée à une administration orale ou vaginale.

4. Forme de dosage en capsule de gélatine molle pharmaceutique selon la revendication 1 ou 2, où la capsule de gélatine molle est destinée à une administration vaginale.

5. Forme de dosage en capsule de gélatine molle pharmaceutique selon l'une quelconque des revendications 1 à 4, où l'acide polyacrylique est le polycarbophile.

6. Forme de dosage en capsule de gélatine molle pharmaceutique selon l'une quelconque des revendications 1 à 5, où l'agent neutralisant est choisi dans le groupe constitué par les C₁-C₆-alkylamines primaires et les C₁-C₆-alkylamines secondaires.

7. Forme de dosage en capsule de gélatine molle pharmaceutique selon l'une quelconque des revendications 1 à 5, où l'agent neutralisant est choisi dans le groupe constitué par la diisopropanolamine, la phénylamine, la glutamine, le chlorure d'hydroxylamine, l'acide p-aminobenzoïque, et les acides aminés choisis parmi la glycine et la lysine.

8. Forme de dosage en capsule de gélatine molle pharmaceutique selon l'une quelconque des revendications 1 à 5, où l'agent neutralisant est une alcanolamine.

9. Forme de dosage en capsule de gélatine molle pharmaceutique selon la revendication 8, où l'alcanolamine est la propanolamine.

10. Forme de dosage en capsule de gélatine molle pharmaceutique selon la revendication 8, où l'alcanolamine est la diisopropanolamine.

11. Forme de dosage en capsule de gélatine molle pharmaceutique selon l'une quelconque des revendications 1 à 10, où la quantité d'agent neutralisant va de 0,05% à 0,5% en poids du poids total du remplissage.

12. Forme de dosage en capsule de gélatine molle pharmaceutique selon la revendication 11, où la quantité d'agent neutralisant est présente selon une quantité allant de 0,075% à 0,400% en poids du poids total du remplissage.

13. Forme de dosage en capsule de gélatine molle pharmaceutique selon la revendication 11 ou 12, où la quantité d'agent neutralisant est présente selon une quantité allant de 0,100% à 0,300% en poids du poids total du remplissage.

14. Forme de dosage en capsule de gélatine molle pharmaceutique selon la revendication 11, où l'agent neutralisant est la diisopropanolamine.

15. Forme de dosage en capsule de gélatine molle pharmaceutique selon la revendication 14, où la quantité de diisopropanolamine est de 0,2% en poids du poids total du remplissage.
